# EUROPEAN PATENT APPLICATION

(11) **EP 4 682 251 A1**
(43) Date of publication of application: **21.01.2026**
(21) Application number: 24774850.2
(22) Date of filing: 15.03.2024
(51) Int. Cl.: C12N 5/10, C12Q 1/02, C12N 15/12

(54) **CELLS DERIVED FROM INSECT OF SUBFAMILY ARCTIINAE**

(30) Priority: 17.03.2023 JP 2023043260
(71) Applicant: Sumitomo Chemical Company, Limited, Osaka-shi, Osaka 541-8550 (JP)
(72) Inventor: SASAOKA, Norio, Osaka-shi, Osaka 554-8558 (JP); TAKAHASHI, Yasuhiko, Osaka-shi, Osaka 554-8558 (JP); KOBAYASHI, Kentaro, Osaka-shi, Osaka 554-8558 (JP)
(74) Representative: Vossius & Partner Patentanwälte Rechtsanwälte mbB
(86) International application number: PCT/JP2024/010191
(87) International publication number: WO 2024/195714

(57) **Abstract**

Provided are cells with excellent refrigeration resistance that can be used in chemical sensors. The cells are derived from an insect of the family *Arctiidae,* containing an exogenous polynucleotide containing a coding sequence for a sensor protein.

## Description

### Technical Field

The present invention relates to cells derived from insects of the family *Arctiidae* and the like.

### Background Art

Groups of odorous substances associated with specific human diseases and mental states have been identified. Owing to their high value as diagnostic markers, there has been growing interest in developing various odor sensors that target these substances. Biological odorant receptors exhibit excellent characteristics, including diversity, sensitivity, and selectivity, which are not found in conventional odor sensor elements such as semiconductors; thus, these odorant receptors offer a promising foundation for the development of novel odor sensors using them as sensor elements.

PTL 1 discloses the use of cells expressing modified odorant receptors or lipid bilayer membranes incorporating modified odorant receptors as odor sensors.

### Citation List

### Patent Literature

PTL 1: WO2022/024902A

### Summary of Invention

### Technical Problem

Odor sensors that rely on the process of artificially preparing lipid bilayer membranes equipped with sensor proteins (e.g., odorant receptors) are often inefficient to produce, and there has been demand for further improvement in the production efficiency of odor sensors. The inventors then focused on using cells that express sensor proteins.

From the perspective of convenience in use, the cells used as a chemical sensor such as an odor sensor are desirably those prepared in advance and retained in containers, which can be used when needed, rather than cells that are cultured and prepared each time for use. In the former case, cells must have storage resistance; it is particularly important that cells have refrigeration resistance from the viewpoint of not drying out the cells.

An object of the present disclosure is to provide cells with excellent refrigeration resistance that can be used in chemical sensors.

### Solution to Problem

In view of the current circumstance described above, the present inventors conducted intensive research and found that cells derived from insects of the family *Arctiidae* have excellent refrigeration resistance and can be used in chemical sensors. The present inventors conducted further research based on this finding and completed the invention of the present disclosure. Specifically, the present disclosure encompasses the following embodiments.

### Item 1.

A cell derived from an insect of the family *Arctiidae,* comprising an exogenous polynucleotide containing a coding sequence for a sensor protein.

### Item 2.

The cell according to Item 1, wherein the sensor protein is an odorant receptor protein.

### Item 3.

The cell according to Item 1 or 2, wherein the sensor protein is an insect odorant receptor protein.

### Item 4.

The cell according to any one of Items 1 to 3, wherein the exogenous polynucleotide comprises a coding sequence for an olfactory receptor co-receptor protein and a coding sequence for a chromogenic or luminescent protein.

### Item 5.

The cell according to any one of Items 1 to 4, wherein the exogenous polynucleotide comprises a coding sequence for a drug-resistant gene.

### Item 6.

The cell according to any one of Items 1 to 5, wherein the exogenous polynucleotide is integrated into genomic DNA of the cell.

### Item 7.

The cell according to any one of Items 1 to 6, wherein the insect of the family *Arctiidae* is an insect of the genus *Spilosoma.*

### Item 8.

The cell according to Item 7, wherein the insect of the genus *Spilosoma* is *Spilosoma imparilis.*

### Item 9.

A cell having refrigeration resistance, comprising an exogenous polynucleotide containing a coding sequence for a sensor protein.

### Item 10.

The cell according to any one of Items 1 to 9, which is determined to have refrigeration resistance when subjected to a refrigeration resistance test, wherein the refrigeration resistance test comprises the following steps:
step (1) of seeding the cell at a density of 1 × 10⁵/200 µL per compartment in a plate with compartments and allowing the cell to stand at 27°C for 6 hours;
step (2) of shielding the plate from light with aluminum foil, allowing the cell to stand at 4°C for 72 hours, and then measuring a fluorescence intensity;
step (3) of adding a substance to which the sensor protein responds to each compartment and then measuring a fluorescence intensity; and
step (4) of determining that the cell has refrigeration resistance when the fluorescence intensity measured in step (3) is greater than the fluorescence intensity measured in step (2).

### Item 11.

The cell according to any one of Items 1 to 10, having a survival rate of 50% or more after storage at 4°C for 15 days.

### Item 12.

A cell chip comprising one or more compartments each containing the cell of any one of Items 1 to 11.

### Item 13.

The cell chip according to Item 12, which is for use in the detection of a chemical substance.

### Advantageous Effects of Invention

The present disclosure provides a cell with excellent refrigeration resistance that can be used in chemical sensors, and a cell chip containing the cell.

### Brief Description of Drawings

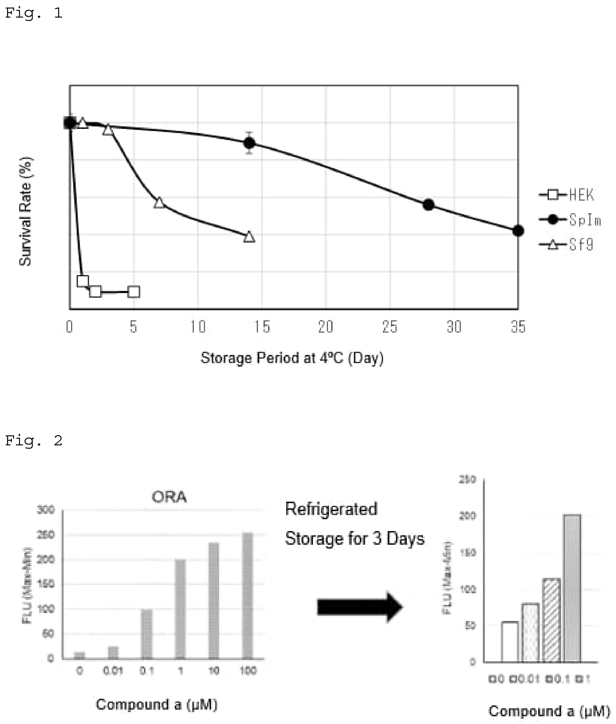

Fig. 1 shows the cell survival rate measured in Test Example 1. The vertical axis represents the cell survival rate (100% at day 0), and the horizontal axis represents the storage period at 4°C. The legend indicates the cells used.
Fig. 2 shows the chemical response activity of ORA cells measured in Test Example 2. The vertical axis represents the difference in fluorescence intensity before and after the addition of a chemical substance (compound a), and the horizontal axis represents the concentration of the chemical substance. The left side of the figure shows the results without refrigerated storage, and the right side of the figure shows the results with refrigerated storage.

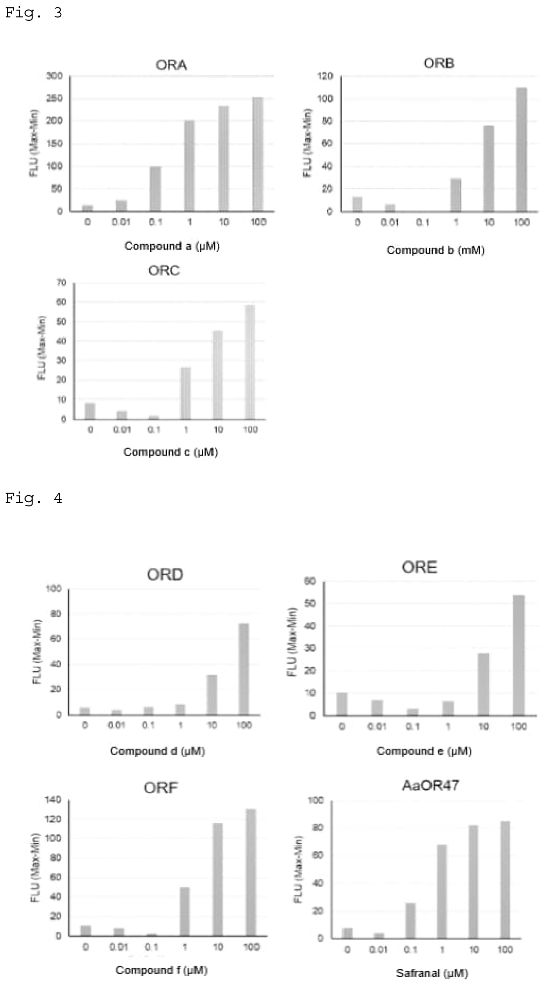

Fig. 3 shows the chemical response activity of SpIm cells stably expressing odorant receptors measured in Test Example 3. The odorant receptor expressed by the cells is indicated above each graph, and the chemical substance used is shown below each graph. The vertical axis represents the difference in fluorescence intensity before and after the addition of a chemical substance, and the horizontal axis represents the concentration of the chemical substance.
Fig. 4 shows the chemical response activity of SpIm cells stably expressing odorant receptors measured in Test Example 3. The odorant receptor expressed by the cells is indicated above each graph, and the chemical substance used is shown below each graph. The vertical axis represents the difference in fluorescence intensity before and after the addition of a chemical substance, and the horizontal axis represents the concentration of the chemical substance.

### Description of Embodiments

In the present specification, the terms "comprising," "containing," and "including" include the concepts of comprising, containing, consisting essentially of, and consisting of.

In an embodiment, the present disclosure relates to a cell derived from an insect of the family *Arctiidae* containing an exogenous polynucleotide that includes a coding sequence for a sensor protein ("the cell of the present disclosure" in the present specification). The cell of the present disclosure is explained below.

The cells derived from insects of the family *Arctiidae* are not particularly limited as long as they are primary cultured cells or established cell lines of biological constituent cells derived from insects of the family *Arctiidae.*

Examples of the family *Arctiidae* include subfamilies such as *Arctiinae, Lithosiinae,* and *Syntominae,* among which the subfamily *Arctiinae* is preferred. The subfamily *Arctiinae* is, for example, preferably the genus *Spilosoma, Spilarctia,* or *Rhagonis,* with the genus *Spilosoma* being particularly preferable. While there is no particular limitation to the genus *Spilosoma, Spilosoma imparilis* is particularly preferable from the perspective of refrigeration resistance and other factors.

The cells derived from insects of the family *Arctiidae* can be obtained from known biological banks or collected and cultured from living insects of the family *Arctiidae* according to or with reference to known methods; if necessary, they can be established as cell lines.

Examples of cells derived from *Spilosoma imparilis* include FFPRI-SpIm-2AM-SF cells (MAFF No.: 275052) and FFPRI-SpIm-2AM-IPL411 cells (MAFF No.: 275053) from the Genebank Project, National Agriculture and Food Research Organization.

The cell of the present disclosure is a cell derived from an insect of the family *Arctiidae* and contains an exogenous polynucleotide that includes a coding sequence for a sensor protein.

The exogenous polynucleotide is not particularly limited as long as it refers to a polynucleotide containing a base sequence that is not derived from the genomic DNA (in particular, chromosomal genomic DNA) of the cell derived from an insect of the family *Arctiidae.*

In the present specification, the polynucleotide includes not only typical polynucleotides such as DNA and RNA inherent in organisms, but also polynucleotides with known chemical modifications, artificial polynucleotides, and like polynucleotides, as listed below. To prevent degradation due to hydrolases such as nucleases, the phosphate residue of each nucleotide can be substituted with a chemically modified phosphate residue, such as phosphorothioate (PS), methylphosphonate, or phosphorodithionate. The hydroxyl group at position 2 of the ribose of each ribonucleotide may also be substituted with -OR (R indicates, for example, CH₃(2'-O-Me), CH₂CH₂OCH₃(2'-O-MOE), CH₂CH₂NHC(NH)NH₂, CH₂CONHCH₃, or CH₂CH₂CN). Additionally, the nucleobase moiety (pyrimidine, purine) may be chemically modified, by, for example, introduction of a methyl group or a cationic functional group into position 5 of the pyrimidine base, or substitution of the carbonyl group at position 2 with thiocarbonyl. Additionally, the polynucleotide of the present invention also includes, but is not limited to, those formed by modifying the phosphate moiety or the hydroxyl moiety, for example, with biotin, an amino group, a lower alkyl amine group, or an acetyl group. The polynucleotide for use can also be, for example, BNA (LNA), which is prepared by crosslinking the 2' oxygen and the 4' carbon in the ribose moiety of a nucleotide to fix the ribose moiety in N-conformation.

The sensor protein may be selected from proteins capable of detecting the presence of a chemical substance, such as a receptor protein that uses a chemical substance as a ligand. The sensor protein is particularly preferably an odorant receptor protein.

The odorant receptor protein is a membrane protein with a seven-transmembrane structure and functions as an odor sensor in organisms. The odorant receptor protein is composed of the following components that are sequentially linked from the amino terminus ("N-terminus" below) to the carboxyl terminus ("C-terminus" below): N-terminal region (NT), first transmembrane domain (TM1), first extracellular loop (EC1), second transmembrane domain (TM2), first intracellular loop (IC1), third transmembrane domain (TM3), second extracellular loop (EC2), fourth transmembrane domain (TM4), second intracellular loop (IC2), fifth transmembrane domain (TM5), third extracellular loop (EC3), sixth transmembrane domain (TM6), third intracellular loop (IC3), seventh transmembrane domain (TM7), and C-terminal region (CT). In the present disclosure, each region is determined by structural prediction using TMpred (K. Hofmann, W. Stoffel, TMbase - a database of membrane spanning proteins segments, Biol. Chem. Hoppe-Seyler, 374 (1993), p. 166, https://embnet.vital-it.ch/software/TMPRED_form.html) (with default conditions).

From the perspective of suitability for chemical detection, the odorant receptor protein is particularly preferably an insect odorant receptor protein. The insect of origin for the insect odorant receptor protein is preferably an insect in the order *Diptera,* such as the family *Culicidae* and the family *Drosophilidae;* an insect in the order *Lepidoptera,* such as the family *Bombycidae;* an insect in the order *Hymenoptera,* such as the family *Apidae;* an insect in the order *Orthoptera,* such as the family *Acrididae;* and an insect in the order *Hemiptera,* such as the family *Cimicidae,* and more preferably an insect in the order *Diptera,* such as the family *Culicidae* and the family *Drosophilidae;* an insect in the order *Orthoptera,* such as the family *Acrididae;* and an insect in the order *Hemiptera,* such as the family *Cimicidae.* Examples of insects in the family *Culicidae* include *Anopheles gambiae, Aedes aegypti,* and *Culex quinquefasciatus.* Examples of insects in the family *Drosophilidae* include *Drosophila melanogaster, Drosophila pseudoobscura,* and *Drosophila virilis.* Examples of insects in the family *Bombycidae* include *Bombyx mori, Bombyx mandarina,* and *Trilocha varians.* Examples of insects in the family *Apidae* include *Apis mellifera, Apis florea, Apis dorsata,* and *Bombus terrestris.* Examples of insects in the family *Acrididae* include *Locusta migratoria.* Examples of insects in the family *Cimicidae* include *Cimex lectularius.*

Specifically, examples of wild-type insect odorant receptor proteins include the following: AaOR1, AaOR2, AaOR4, AaOR5, AaOR6, AaOR7, AaOR8, AaOR9, AaOR10a, AaOR15, AaOR22, AaOR24, AaOR25, AaOR26, AaOR27, AaOR28, AaOR30, AaOR34, AaOR36, AaOR38, AaOR41a, AaOR41b, AaOR42, AaOR43, AaOR44, AaOR47, AaOR49, AaOR50, AaOR52, AaOR54, AaOR58, AaOR59, AaOR60, AaOR61, AaOR64, AaOR65, AaOR66, AaOR67a, AaOR69a, AaOR70, AaOR71, AaOR72a, AaOR73, AaOR74, AaOR75, AaOR77, AaOR78, AaOR79, AaOR81, AaOR83b, AaOR84, AaOR85, AaOR86, AaOR87, AaOR91, AaOR95, AaOR97, AaOR96, AaOR99, AaOR100, AaOR102, AaOR103, AaOR104a, AaOR105, AaOR107, AaOR108, AaOR109, AaOR110, AaOR112, AaOR114, AaOR116, AaOR117, AaOR118, AaOR122, AaOR125, AaOR128, AgOR1, AgOR2, AgOR3, AgOR4, AgOR5, AgOR6, AgOR7, AgOR8, AgOR9, AgOR10, AgOR11a, AgOR12a, AgOR12b, AgOR13, AgOR14, AgOR15, AgOR16a, AgOR17, AgOR18, AgOR20, AgOR21, AgOR23, AgOR25, AgOR26, AgOR27, AgOR28, AgOR30, AgOR34, AgOR36, AgOR37, AgOR38, AgOR39a, AgOR40, AgOR42, AgOR44, AgOR45, AgOR46, AgOR47, AgOR49, AgOR50, AgOR54, AgOR56a, AgOR57, AgOR60, AgOR61, AgOR62, AgOR63, AgOR64, AgOR65, AgOR69, AgOR70, AgOR71, AgOR72, AgOR74, AgOR75, AgOR76a, AmOR1, AmOR3, AmOR9, AmOR10, AmOR13, AmOR41, AmOR51, AmOR52, AmOR55, AmOR71, AmOR73, AmOR78, AmOR85, AmOR89, AmOR90, AmOR114, AmOR115, AmOR118, AmOR120, AmOR121, AmOR161, BmOR1, BmOR2, BmOR3, BmOR4, BmOR5, BmOR8, BmOR9, BmOR10, BmOR13, BmOR17, BmOR18, BmOR23, BmOR24, BmOR25, BmOR35, BmOR36, BmOR42, BmOR45, BmOR49, BmOR51, BmOR52, BmOR55, BmOR56, BmOR61, DmOR1a, DmOR9a, DmOR19a, DmOR22a, DmOR22b, DmOR22c, DmOR24a, DmOR30a, DmOR33a, DmOR33b, DmOR33c, DmOR35a, DmOR42b, DmOR43a, DmOR45a, DmOR45b, DmOR47a, DmOR49b, DmOR59b, DmOR65b, DmOR65c, DmOR67b, DmOR67c, DmOR69a, DmOR71a, DmOR74a, DmOR82a, DmOR83a, DmOR83b, DmOR83c, DmOR85a, DmOR85c, DmOR85e, DmOR85f, DmOR88a, DmOR92a, DmOR94a, DmOR94b, and DmOR98b.

In the present specification, OR denotes "odorant receptor." Dm indicates derivation from *Drosophila melanogaster,* Bm indicates derivation from *Bombyx mori,* Ag indicates derivation from *Anopheles gambiae,* and Aa indicates derivation from *Aedes aegypti.* The amino acid sequences of various odorant receptor proteins, including these, and their coding sequences are either known or can be readily identified through sequence identity searches based on known sequences.

The sensor protein may contain one or more amino acid mutations in its wild-type amino acid sequence, provided that the chemical response activity is not significantly decreased. The phrase "not significantly decreased" means, for example, that the chemical response activity of a sensor protein containing amino acid mutations is, for example, at least 50%, preferably at least 60%, more preferably at least 70%, even more preferably at least 80%, and yet more preferably at least 90% of the chemical response activity of the wild-type sensor protein, taken as 100%.

The amino acid mutation is, for example, substitution, insertion, addition, or deletion of an amino acid, preferably substitution, and particularly preferably conservative substitution.

In the present specification, "conservative substitution" refers to the substitution of an amino acid residue with another amino acid residue having a similar side chain. For example, the substitution between amino acid residues having a basic side chain such as lysine, arginine, or histidine is considered to be a conservative substitution. The following substitutions between other amino acid residues are also considered to be a conservative substitution: the substitution between amino acid residues having an acidic side chain such as aspartic acid and glutamic acid; the substitution between amino acid residues having an uncharged polar side chain such as glycine, asparagine, glutamine, serine, threonine, tyrosine, or cysteine; the substitution between amino acid residues having a nonpolar side chain such as alanine, valine, leucine, isoleucine, proline, phenylalanine, methionine, or tryptophan; the substitution between amino acid residues having a β-branched side chain such as threonine, valine, or isoleucine; and the substitution between amino acid residues having an aromatic side chain such as tyrosine, phenylalanine, tryptophan, or histidine.

The sensor protein may contain its wild-type amino acid sequence or an amino acid sequence having, for example, at least 70%, preferably at least 80%, more preferably at least 90%, even more preferably at least 95%, still more preferably at least 98%, and particularly preferably at least 99% identity to the wild-type amino acid sequence.

The "identity" of amino acid sequences refers to the degree to which two or more contrastable amino acid sequences match each other. Thus, the higher the degree of match between two amino acid sequences, the higher the identity or similarity of those sequences. The level of amino acid sequence identity is determined, for example, by using FASTA, which is a tool for sequence analysis, with default parameters. Alternatively, the level of amino acid sequence identity can be determined by using the BLAST algorithm by Karlin and Altschul (Karlin S., Altschul S. F. Methods for assessing the statistical significance of molecular sequence features by using general scorings schemes, Proc. Natl. Acad. Sci. USA. 87: 2264-2268 (1990); Karlin S., Altschul S. F. Applications and statistics for multiple high-scoring segments in molecular sequences, Proc Natl Acad Sci USA. 90: 5873-7 (1993)). A program called "BLASTX," based on this BLAST algorithm, has been developed. The specific techniques of these analysis methods are known and can be found on the website of the National Center of Biotechnology Information (NCBI) (http://www.ncbi.nlm.nih.gov/).

The sensor protein may have other amino acid sequences, such as protein tags, fluorescent proteins, luminescent proteins, signal sequences, or other proteins or peptides, attached thereto, provided that the chemical response activity is not significantly impaired. Examples of protein tags include biotin, His tag, FLAG tag, Halo tag, MBP tag, HA tag, Myc tag, V5 tag, and PA tag.

In the present specification, chemical response activity refers to the property of a sensor protein to recognize a chemical substance and initiate signal transduction activity (e.g., ion channel activity) either alone or in conjunction with other proteins. In the case of odorant receptors, chemical response activity refers to the property of an odorant receptor to recognize a chemical substance and form an odorant receptor complex, together with an olfactory receptor co-receptor, thereby exhibiting ion channel activity. The chemical response activity of a sensor protein can be measured by using the signal transduction activity of the sensor protein in contact with a chemical substance as an indicator (e.g., by quantifying and evaluating the amount of signal molecules). In the case of odorant receptors, their chemical response activity can be measured using the ion channel activity of an odorant receptor complex formed of an odorant receptor in contact with a chemical substance and an olfactory receptor co-receptor as an indicator. For example, a chemical substance is brought into contact with cells that express a protein that becomes colored or luminescent in response to the influx of ions (e.g. calcium ions) into the cells when (a) an odorant receptor, (b) an olfactory receptor co-receptor, and (c) an odorant receptor complex respond, and the amount of luminescence of the cells is measured. The greater the measured amount of luminescence, the higher the response activity of the odorant receptor to the chemical substance is determined to be. Specifically, the chemical response activity can be measured according to the method described in PTL 1.

The coding sequence for the sensor protein is not particularly limited as long as it is a base sequence encoding the sensor protein. In an embodiment, the exogenous polynucleotide contains an expression cassette for a sensor protein. The expression cassette is not particularly limited as long as it is a polynucleotide capable of expressing the sensor protein within a cell. A typical example of expression cassettes for a sensor protein is a polynucleotide containing a promoter and the coding sequence for the sensor protein placed under control of the promoter.

The promoter is not particularly limited and can be selected as appropriate. The promoter for use can be selected from various types of pol II promoters, for example. Examples of pol II promoters include, but are not particularly limited to, CMV promoter, EF1 promoter, SV40 promoter, MSCV promoter, and promoters derived from insect genes.

When the sensor protein is an insect odorant receptor, it is preferred that the exogenous polynucleotide contain a coding sequence for an insect olfactory receptor co-receptor, from the viewpoint that the cell of the present disclosure can be used directly in the detection of a chemical substance. The insect olfactory receptor co-receptor is a membrane protein with a seven-transmembrane structure as with the odorant receptor. However, the olfactory receptor co-receptor itself does not recognize odorant substances, but functions by forming a hetero-complex with an odorant receptor. The odorant receptor complex, which is a hetero-complex composed of an odorant receptor and an olfactory receptor co-receptor, has ion channel activity that is activated by odorant substances. When activated, the odorant receptor complex allows the influx of cations such as sodium ions (Na⁺) and calcium ions (Ca²⁺) into cells.

From the viewpoint that the cell of the present disclosure can be used directly in the detection of a chemical substance, it is preferred that the exogenous polynucleotide include a coding sequence for a protein that becomes colored or luminescent in response to the influx of ions (e.g., calcium ions) into the cell when the sensor protein (in particular, the odorant receptor protein) responds. Examples of such proteins include aequorin, Yellow Cameleon (YC), and GCaMP. Alternatively, the cell of the present disclosure preferably contains an ion-dependent fluorescent dye, such as calcium ion-dependent fluorescent dyes (e.g., Fura-2, Fluo-3, and Fluo-4).

From the viewpoint that the cell of the present disclosure can be used directly in the detection of a chemical substance, the cell of the present disclosure contains a sensor protein; i.e., a sensor protein is expressed in the cell of the present disclosure. In an embodiment, the sensor protein has a seven-transmembrane structure and is thus positioned on the cell membrane as a membrane protein.

The exogenous polynucleotide preferably contains a coding sequence for a drug-resistant gene to allow for drug screening of the cell of the present disclosure. For the drug-resistant gene, a gene resistant to a drug that can be used for drug screening of insect cells may be selected. Examples of drug-resistant genes include chloramphenicol resistance gene, tetracycline resistance gene, neomycin resistance gene, erythromycin resistance gene, spectinomycin resistance gene, kanamycin resistance gene, hygromycin resistance gene, and puromycin resistance gene.

It is preferred that the exogenous polynucleotide contains coding sequences such as a coding sequence for an insect olfactory receptor co-receptor, a coding sequence for a chromogenic or luminescent protein, and a coding sequence for a drug-resistant gene in the form of an expression cassette. The structure of the expression cassette is similar to that of the expression cassette for the sensor protein. The promoter of the expression cassette can be shared among multiple coding sequences.

The exogenous polynucleotide is preferably integrated into the genomic DNA (particularly preferably chromosomal genomic DNA). This enables stable expression of the sensor protein, making it suitable for chemical detection. In this case, the exogenous polynucleotide can be a single continuous region within the genomic DNA or a combination of two or more continuous regions (e.g., a form in which the sensor protein-coding sequence is included in continuous region A, and the coding sequence for a drug-resistant gene is included in continuous region B that is separate from continuous region A, or a form in which the sensor protein-coding sequence is included in both continuous region A and continuous region B).

In another embodiment, the exogenous polynucleotide may be in a state in which it is not integrated into the genomic DNA. In this case, the exogenous polynucleotide can be in the form of a vector, for example. In this case, the exogenous polynucleotide can be a single polynucleotide molecule, or two or more polynucleotide molecules (e.g., a form in which the sensor protein-coding sequence is included in polynucleotide molecule A, while the coding sequence for a drug-resistant gene is included in polynucleotide molecule B, which is a separate molecule from polynucleotide molecule A, or a form in which the sensor protein-coding sequence is included in both polynucleotide molecule A and polynucleotide molecule B).

In an embodiment, the present disclosure relates to a cell with refrigeration resistance, comprising an exogenous polynucleotide containing a coding sequence for a sensor protein ("the cell of the present disclosure").

In an embodiment of the present invention, the refrigeration resistance can be determined based on the following refrigeration resistance test 1:
Step (1) of seeding cells at a density of 1 × 10⁵/200 µL per compartment in a plate with compartments, and allowing the cells to stand at 27°C for 6 hours;
Step (2) of shielding the plate from light with aluminum foil, allowing the cells to stand at 4°C for 72 hours, and then measuring a fluorescence intensity;
Step (3) of adding a substance to which the sensor protein responds to each compartment, and then measuring a fluorescence intensity; and
Step (4) of determining that the cells have refrigeration resistance when the fluorescence intensity measured in step (3) is greater than the fluorescence intensity measured in step (2).

For more detailed conditions of refrigeration resistance test 1, refer to Test Example 2, described below.

In an embodiment of the present invention, the refrigeration resistance can be defined as a survival rate of a certain level or higher after storage at 4°C for a predetermined period. For example, the refrigeration resistance can be a survival rate of, for instance, 50% or higher, preferably 60% or higher, more preferably 70% or higher, and even more preferably 80% or higher after storage at 4°C for 14 days. The refrigeration resistance is measured according to Test Example 1, described below.

From the perspective of suitability for use in chemical sensors, the cell of the present disclosure is preferably incorporated in a cell chip. Thus, in an embodiment, the present disclosure relates to a cell chip including one or more compartments each containing the cell of the present disclosure ("the cell chip of the present disclosure" in the present specification).

The compartments of the cell chip of the present disclosure preferably contain cells and hydrogel. This protects the cells from desiccation.

The configuration of the compartments is not particularly limited as long as it is capable of retaining the cells. From the perspectives of cell retention, production efficiency, or chemical detection, the compartments are preferably in the form of wells.

The material of the compartments is not particularly limited as long as the material can retain cells. The material can be, for example, resin or metal.

From the perspective of detection sensitivity, a compartment typically contains multiple cells. The number of cells per unit area (cm²) in a compartment is, for example, 1×10³ to 1×10⁹ cells/cm².

From the perspective of detection sensitivity or production efficiency, the bottom surface area of a single compartment is preferably 0.5 to 100 mm², more preferably 1 to 30 mm², and even more preferably 1.5 to 10 mm².

From the perspective of detection sensitivity or production efficiency, the number of compartments included in the cell chip is preferably 10 to 2000, more preferably 30 to 1000, and even more preferably 50 to 500.

The cell of the present disclosure has excellent refrigeration resistance. In the present specification, "refrigeration resistance" means that the cell survival rate and/or chemical response activity is maintained at a higher level after a predetermined period (e.g., 1 day or more, 1 to 30 days, 2 to 20 days, or 3 to 10 days) of refrigerated storage (e.g., 0 to 10°C, 0 to 8°C, 0 to 6°C, or 2 to 6°C). The cell survival rate and chemical response activity can be measured according to or with reference to the methods described in the Examples below.

The cell of the present disclosure exhibits excellent chemical response activity by using a sensor protein as compared to cells derived from other species. The cell of the present disclosure is also suitable for detecting chemical substances using various sensor proteins.

The cell of the present disclosure and the cell chip of the present disclosure can be used in the detection of chemical substances (in particular, odorous substances). The chemical substances can be, for example, those in a sample such as body fluids (e.g., urine, blood, and saliva), air (e.g., indoor air and air inside packaging), and water (e.g., river water, seawater, tap water, clean water, and sewage). In this case, for example, the cell of the present disclosure is brought into contact with the sample, or the sample is added to compartments of the cell chip of the present disclosure; this allows the chemical substance in the sample to reach the cells and come into contact with the sensor protein in the cells. For example, a chemical substance can be detected by detecting ions flowing into a cell (e.g., by detecting a protein that becomes colored or luminescent in response to ions).

### Examples

The present invention will be described in detail below with reference to Examples. However, the present invention is not limited to these Examples.

### Test Example 1: Refrigeration Resistance Test 1

Cells derived from *Spilosoma imparilis* (SpIm cells), Sf9 cells, and HEK293 cells were individually seeded in the wells of a 96-well plate at 3 × 10³ cells/well with n = 3. The number of cells at the time of seeding was measured by staining the cell suspension with trypan blue and using a Countess II FL Automated Cell Counter (Thermo Fisher). The culture media used were as follows: SpIm cells: Sf-900III SFM medium; Sf9 cells: Sf-900III SFM medium; HEK293 cells: Dulbecco's Modified Eagle's Medium (4.5 g/L glucose) (containing L-glutamine and HEPES, without pyruvic acid) .

After cell seeding, the 96-well plate was shielded from light with aluminum foil and placed in a refrigerator (4°C). Cell survival rate was measured according to the MTT assay (Roche kit) at 14, 28, and 35 days for SpIm cells, at 1, 3, 7, and 14 days for Sf9 cells, and at 1, 2, and 5 days for HEK293 cells after the start of static culture.

The MTT assay was conducted according to the procedure manual included in the kit as follows. After an MTT solution was added at 10 µL/well, the cells were left to stand at 27°C for 4 hours. Subsequently, an MTT eluate was added at 100 µL/well, and the cells were left to stand overnight in a 37°C incubator. Subsequently, the absorbance at 560 nm was measured using a TECAN Infinite M200 PRO reader. The cell survival rate (%) was calculated by dividing the absorbance of the post-seeding sample by the absorbance of the sample at the time of seeding (day 0).

Fig. 1 shows the results. SpIm cells were found to have higher survival rates even when stored for an extended period of time under refrigerated conditions.

### Test Example 2: Refrigeration Resistance Test 2

### 2-1. Preparation of Stably Expressing Cell

SpIm cells were transfected with a transposon vector containing the coding sequence for an odorant receptor protein (ORA), the coding sequence for an olfactory receptor co-receptor, the coding sequence for a calcium sensor luminescence protein, and the coding sequence for a puromycin resistance gene that were all placed under control of a promoter sequence and that were positioned between the 5' ITR (inverted terminal repeat) and 3' ITR. Selection was performed with puromycin, thereby preparing SpIm cells stably expressing odorant receptors in which the foreign DNA containing the above coding sequences and promoter sequence was integrated into the chromosomal genomic DNA ("ORA cells" below)

### 2-2. Measurement of Sensor Functionality

ORA cells were seeded in a 96-well plate (CORNING 3909) at a density of 1 × 10⁵ cells/200 µL/well and left to stand in a 27°C incubator (without CO₂ supply). The culture medium used was Sf-900III SFM medium. Cell counting was performed using a Countess II FL Automated Cell Counter (Thermo Fisher) after the cell suspension was stained with trypan blue. After 24 hours from seeding, the culture fluid was removed from the 96-well plate and replaced with 80 mL of 0.1% BSA HBSS (-) (without phenol red). After five minutes from this fluid exchange operation, the fluorescence intensity (GCaMP) was measured using a FlexStation 3 microplate reader. Specifically, compound a (substances to which ORAs respond) adjusted to various concentrations (0, 0.01, 0.1, 1, 10, and 100 µM) was added to the wells, and changes in fluorescence intensity before and after addition of compound a were quantified using a microplate reader (FlexStation3, Molecular Devices).

### 2-3. Measurement of Sensor Functionality after Refrigerated Storage

After being seeded in the same manner as in section 2-2 above, the ORA cells were left to stand in a 27°C incubator (without CO₂ supply) for 6 hours, then shielded from light with aluminum foil, and then left in a refrigerator (4°C). After 72 hours, the fluorescence intensity before and after the addition of compound a was measured in the same manner as in section 2-2 above.

### 2-4. Results

Fig. 2 shows the results. SpIm cells were found to be usable as a chemical response sensor, and their sensor functionality was found to remain stable even after refrigerated storage.

### Test Example 3: Chemical Response Activity Evaluation Test

Splm cells stably expressing odorant receptor proteins were created for each odorant receptor protein (ORA, ORB, ORC, ORD, ORE, ORF, and AaOR47) in the same manner as in section 2-1 above using Splm cells. The chemical response activity was evaluated using each type of these cells in the same manner as in section 2-2 above.

Figs. 3 and 4 show the results. SpIm cells were found to show chemical response activity even when various odorant receptors were used.

## Claims

1. A cell derived from an insect of the family *Arctiidae,* comprising an exogenous polynucleotide containing a coding sequence for a sensor protein.

2. The cell according to claim 1, wherein the sensor protein is an odorant receptor protein.

3. The cell according to claim 1, wherein the sensor protein is an insect odorant receptor protein.

4. The cell according to claim 1, wherein the exogenous polynucleotide comprises a coding sequence for an olfactory receptor co-receptor protein and a coding sequence for a chromogenic or luminescent protein.

5. The cell according to claim 1, wherein the exogenous polynucleotide comprises a coding sequence for a drug-resistant gene.

6. The cell according to claim 1, wherein the exogenous polynucleotide is integrated into genomic DNA of the cell.

7. The cell according to claim 1, wherein the insect of the family *Arctiidae* is an insect of the genus *Spilosoma.*

8. The cell according to claim 7, wherein the insect of the genus *Spilosoma* is *Spilosoma imparilis.*

9. A cell having refrigeration resistance, comprising an exogenous polynucleotide containing a coding sequence for a sensor protein.

10. The cell according to claim 9, which is determined to have refrigeration resistance when subjected to a refrigeration resistance test, wherein the refrigeration resistance test comprises the following steps:
step (1) of seeding the cell at a density of 1 × 10⁵/200 µL per compartment in a plate with compartments and allowing the cell to stand at 27°C for 6 hours;
step (2) of shielding the plate from light with aluminum foil, allowing the cell to stand at 4°C for 72 hours, and then measuring a fluorescence intensity;
step (3) of adding a substance to which the sensor protein responds to each compartment and then measuring a fluorescence intensity; and
step (4) of determining that the cell has refrigeration resistance when the fluorescence intensity measured in step (3) is greater than the fluorescence intensity measured in step (2).

11. The cell according to claim 9 or 10, having a survival rate of 50% or more after storage at 4°C for 15 days.

12. A cell chip comprising one or more compartments each containing the cell of any one of claims 1 to 11.

13. The cell chip according to claim 12, which is for use in the detection of a chemical substance.
